# EUROPÄISCHE PATENTSCHRIFT

(11) **EP 0 897 713 B1**
(45) Veröffentlichungstag und Bekanntmachung des Hinweises auf die Patenterteilung: **02.04.2003**
(21) Anmeldenummer: 98115302.6
(22) Anmeldetag: 14.08.1998
(51) Int. Cl.: A61K 7/13

(54) **Haarfärbemittel**
Hair-dye
Teinture pour cheveux

(30) Priorität: 19.08.1997 DE 19735872
(43) Veröffentlichungstag der Anmeldung: 24.02.1999
(73) Patentinhaber: KPSS-Kao Professional Salon Services GmbH, 64297 Darmstadt (DE)
(72) Erfinder: Lorenz, Heribert, 64401 Gross-Bieberau (DE); Kufner, Frank, 64297 Darmstadt (DE)

(56) Entgegenhaltungen:
- EP-A- 0 782 845
- DE-A- 3 521 995

## Beschreibung

Die vorliegende Erfindung betrifft ein Haarfärbemittel auf Basis eines mit Peroxid reagierenden Oxidationsfarbstoffvorprodukt-Systems, das ausdrucksvolle und dauerhafte Farbtöne im Blau- und Violett-Bereich liefert, die entweder als solche angewandt werden, oder, in Kombination mit weiteren Entwicklerund/oder Kupplersubstanzen, zur Erzielung weiterer Farbnuancen benutzt werden können.

Es sind bereits zahlreiche Entwickler/Kuppler-Kombinationen bekannt, die zu den verschiedensten Haarfärbungen führen. Gleichwohl besteht nach wie vor ein Bedürfnis nach Haarfärbemitteln, die nicht nur in toxikologischer und dermatologischer Hinsicht absolut unbedenklich sind, sondern auch die Herstellung farbtechnisch anspruchsvoller dauerhafter Haarfärbungen, insbesondere im Blau- und Violettbereich, ermöglichen.

Diese Aufgabe wird dadurch gelöst, daß ein solches Haarfärbemittel ein mit Peroxid reagierendes Oxidationsfarbstoff-Vorprodukt enthält, das aus mindestens einer Entwicklersubstanz ausgewählt aus der Gruppe 1,4-Diaminobenzol, 2,5-Diaminotoluol, 2-(2,5-Diaminophenyl)ethanol,2-(2'-Hydroxyethylamino)-5-aminotoluol, N,N-Bis-(hydroxyethyl)-1,4-diaminobenzol, 3-(N,N-Diethylaminomethyl)-4-hydroxyanilin, 4-Aminophenol, 2-Chlor-4-aminophenol, 2-Amino-5-N,N-diethylaminotoluol und/oder 4-Amino-3-methylphenol bzw. deren wasserlöslichen Salzen, und 3-Amino-N,N-dimethylanilin bzw. dessen wasserlöslichen Salzen besteht.

Bei Anwendung dieser Zusammensetzungen auf Basis einer üblichen Grundlage werden nach der Oxidation mit Peroxid sehr ausdrucksvolle Grundfärbungen, insbesondere im Blau- bzw. Violett-Bereich, erhalten, die durch Einsatz von Gemischen entsprechender Kupplersubstanzen zu anderen Farbnuancen variiert werden können.

Die bevorzugten, zusätzlich einsetzbaren Kupplersubstanzen, die erfindungsgemäß vorzugsweise in einem molaren Verhältnis von Entwicklersubstanz zu Kupplersubstanz zwischen etwa 1 : 2 und etwa 5 : 1,vorzugsweise etwa 1 :1 bis etwa 2,5 : 1, verwendet werden, sind Resorcin, 2-Methylresorcin, 4-Chlorresorcin, 2-Amino-4-chlorphenol, 4-(N-Methyl)aminophenol, 2-Aminophenol, 3-Aminophenol, 1-Methyl-2-hydroxy-4-aminobenzol, 3-N,N-Dimethylaminophenol, 5-Amino-2-methylphenol, 6-Amino-3-methyl-phenol, 3-Amino-2-methylamino-6-methoxypyridin, 2-Amino-3-hydroxypyridin, 4-Aminodiphenylamin, 4,4'-Diaminodiphenylamin, 2-Dimethylamino-5-aminopyridin, 2,5-Diaminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, 1,3-Diaminotoluol, α-Naphthol, 1,4-Diamino-2-chlorbenzol, 4,6-Dichlorresorcin, 4-Hydroxy-1,2-methylendioxybenzol, 1,5-Dihydroxynaphthalin, 1,7-Dihydroxynaphthalin, 2,7-Dihydroxynaphthalin, 1-Hydroxynaphthalin, 4-Hydroxy-1,2-methylendioxybenzol, 2,4-Diamino-3-chlorphenol, und/oder 1-Methoxy-2-amino-4-(2'-hydroxyethylamino)benzol.

Bevorzugte zusätzliche Kupplersubstanzen sind insbesondere Resorcin, 2-Methylresorcin, 4-Chlorresorcin, 3-Aminophenol, 1-Methyl-2-hydroxy-4-aminobenzol, 2-Amino-4-β-hydroxyethylaminoanisol bzw. dessen wasserlösliche Salze und α-Naphthol.

Damit soll jedoch der Zusatz weiterer Kupplersubstanzen keineswegs ausgeschlossen sein.

Auch die Mitverwendung weiterer, an sich bekannter Entwicklersubstanzen ist, möglich, beispielsweise, 5-Aminosalicylsäure und/oder weiterer Hydroxytriaminopyrimidine, wie sie aus der EP-B 467 026 bekannt sind.
Als solche werden insbesondere das aus der bereits erwähnten EP-B 467 026 bekannte 4-Hydroxy-2,5,6-triaminpyrimidin, 2,4,5-Triamino-6-hydroxypyrimidin und das 4,5,6-Triamino-2-hydroxypyrimidin eingesetzt, vorzugsweise als Sulfat-Salze.

Die Konzentration der Entwicklersubstanzen liegt zwischen etwa 0,05 und 5 %, vorzugsweise 0,1 und 4 %, insbesondere 0,25 bis 0,5 % und 2,5 bis 3 % Gew.-% der Gesamtzusammensetzung des Haarfärbemittels (ohne Oxidationsmittel), wobei sich die Angaben jeweils auf den Anteil an freier Base beziehen.

Die Kupplersubstanzen als Reaktionspartner der Entwicklersubstanz(en) liegen in den erfindungsgemäßen Haarfärbemitteln normalerwiese etwa im gleichen Anteil wie die Entwicklersubstanzen vor, d. h., also in Mengen von 0,05 bis 5,0 %, vorzugsweise 0,1 bis 4 %, insbesondere 0,5 bis 3 Gew.-% der Gesamtzusammensetzung (ohne Oxidationsmittel), wobei sich die Angaben jeweils auf den Anteil an freier Base beziehen.

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel). Das 3-Amino-N,N-dimethylanilin gelangt vorzugsweise in Form seiner wasserlöslichen Salze, beispielsweise als Dihydrochlorid oder Sulfat, zum Einsatz.

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind. Sie können als Lösungen, Cremes, Gele oder auch in Form von Aerosol-Präparaten vorliegen; geeignete Trägermaterial-Zusammensetzungen sind aus dem Stand der Technik hinreichend bekannt.

Zur Applikation wird das erfindungsgemäße Oxidationsfarbstoff-Vorprödukt mit einem Oxidationsmittel vermischt. Bevorzugtes Oxidationsmittel ist Wasserstoffperoxid beispielsweise in 2- bis 6-prozentiger Konzentration.

Es können jedoch auch andere Peroxide wie Harnstoffperoxid und Melaminperoxid eingesetzt werden.

Im Prinzip ist auch eine Oxidation mit Luftsauerstoff möglich. Der pH-Wert des applikationsfertigen Haarfärbemittels, d. h. nach Vermischung mit Peroxid, kann sowohl im schwach sauren, d. h. einem Bereich von 5,5 bis 6,9, im neutralen als auch im alkalischen Bereich, d. h. zwischen pH 7,1 und 10,5 liegen.

| Grundlage | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Natriumsulfit | 1,0 |
| Eiweißhydrolysat | 0,5 |
| Ascorbinsäure | 0,2 |
| Parfum | 0,4 |
| Ammoniak, 25%ig | 8,5 |
| Ammoniumchlorid | 0,5 |
| Panthenol | 0,8 |
| Wasser | @ 100,00 |

Die erfindungsgemäße Entwickler-Kuppler-Kombination wurde, unter entsprechender Verringerung des Wassergehalts, in diese Grundlage eingearbeitet.

Die Ausfärbungen erfolgen jeweils an Woll-Läppchen und Strähnen aus gebleichtem Menschenhaar, durch Aufbringen einer einem alkalischen pH-Wert aufweisenden Mischung aus Farbstoff-Vorprodukt und 6%-iger Wasserstoffperoxid-Lösung und zwanzigminütiger Einwirkung bei Zimmertemperatur, Auswaschen und Trocknen.

Es wurden die folgenden Färbungen erzielt:

| | | |
|---|---|---|
| Beispiel 1 | 0,50 (Gew.-%) | 2,5 Diaminotoluolsulfat |
| | 0,47 | 3-Amino-N,N-diethylanilindihydrochlorid (PC 64) |
| Färbung | Intensives Dunkeltintenblau. | |

| | | |
|---|---|---|
| Beispiel 2 | 0,24 (Gew.-%) | 1,4 Diaminobenzol |
| | 0,47 | PC 64 |
| Färbung | Intensives Tiefblau. | |

| | | |
|---|---|---|
| Beispiel 3 | 0,56 (Gew.-%) | 2-(2,5-Diaminophenyl)ethanol |
| | 0,25 | PC 64 |
| Färbung | Intensives Dunkelblau. | |

| | | |
|---|---|---|
| Beispiel 4 | 0,71 (Gew.-%) | 2-(2'-Hydroxyethylamino)-5-toluolsulfat . 3 H₂O |
| | 0,47 | PC 64 |
| Färbung | Intensives Blau. | |

| | | |
|---|---|---|
| Beispiel 5 | 0,33 (Gew.-%) | 2-Chlor-4-aminophenol |
| | 0,47 | PC 64 |
| Färbung | Intensives Dunkel-Blauviolett. | |

| | | |
|---|---|---|
| Beispiel 6 | 0,66 (Gew.-%) | N,N-Bishydroxyethyl-1,4-diaminobenzolsulfat |
| | 0,47 | PC 64 |
| Färbung | Intensives Azurblau. | |

| | | |
|---|---|---|
| Beispiel 7 | 0,25 (Gew.-%) | 4-Aminophenol |
| | 0,47 | PC 64 |
| Färbung | Intensives Violett. | |

| | | |
|---|---|---|
| Beispiel 8 | 0,48 (Gew.-%) | 2-Amino-5-N,N-diethylaminotoluolhydrochlorid |
| | 0,67 | PC 64 |
| Färbung | Intensive Taubenblau. | |

| | | |
|---|---|---|
| Beispiel 9 | 0,60 (Gew.-%) | 3-(N,N-Diethylaminomethyl)-4-4-hydroxyanilindihydrochlorid |
| | 0,47 | PC 64 |
| Färbung | Intensives Kupferviolett. | |

| | | |
|---|---|---|
| Beispiel 10 | 0,28 (Gew.-%) | 4-Amino-3-methylphenol |
| | 0,47 | PC 64 |
| Färbung | Intensives Blaurotviolett. | |

## Patentansprüche

1. Haarfärbemittel auf Basis eines mit Peroxid oxidierbaren Entwickler-Kuppler-Systems, enthaltend
a)mindestens eine Entwicklersubstanz, ausgewählt aus 1,4-Diaminobenzol,2,5-Diaminotoluol, 2-(2,5-Diaminophenyl)ethanol, 2-(2'-Hydroxyethylamino)-5-aminotoluol, N,N-Bis-(hydroxyethyl)-1,4-diaminobenzol, 3-(N,N-Diethylaminomethyl)-4-hydroxyanilin, 4-Aminophenol, 2-Chlor-4-aminophenol, 2-Amino-5-N,N-diethylaminotoluol und/oder 4-Amino-3-methylphenol bzw. deren wasserlöslichen Salzen, und
b)3-Amino-N,N-dimethylanilin bzw. dessen wasserlösliche Salze.

2. Haarfärbemittel nach Anspruch 1, **dadurch gekennzeichnet, daß** das molare Verhältnis von a) zu b) zwischen 1:2 und 5:1 lilegt.

## Claims

1. Hair dyeing composition based on a developer-coupler system which may be oxidized by peroxide comprising
a) at least one developing substance selected from 1.4-diaminobenzene, 2.5-diaminotoluene, 2-(2.5-diaminophenyl)ethanol, 2-(2'-hydroxyethyl amino)-5-aminotoluene. N.N-bis-(hydroxyethyl)-1.4-diaminobenzene, 3-(N,N-diethylamino methyl)-4-hydroxy aniline, 4-aminophenol, 2-chloro-4-aminophenol. 2-amino-5-N,N-diethyl aminotoluene and/or 4-amino-3-methyl phenol or the water-soluble salts thereof, and
b) 3-amino-N,N-dimethyl aniline or the water-soluble salts thereof.

2. Hair dyeing composition according to claim 1, wherein the molecular ratio of a) to b) is between 1:2 and 5:1.

## Revendications

1. Agent de coloration des cheveux à base d'un système de développement et de couplage par oxydation avec un peroxyde, qui contient:
a) au moins une substance de développement sélectionnée parmi le 1,4 -diaminobenzène, le 2,5-diaminotoluène, le 2 -(2,5-diaminophényl)éthanol, le 2-(2'-hydroxyéthylamino)-5-aminotoluène, le N,N -Bis-(hydroxyéthyl)-1,4-diaminobenzène, la 3 -(N,N-diéthylaminométhyl)-4-hydroxyaniline, le 4 -aminophénol, le 2 -chloro-4-aminophénol, le 2 -amino-5-N,N-diéthylaminotoluène et/ou le 4 -amino-3-méthylphénol ou leurs sels solubles dans l'eau, et
b) de la 3 -amino-N,N-dimithylaniline ou ses sels solubles dans l'eau.

2. Agent de coloration des cheveux selon la revendication 1, **caractérisé en ce que** le rapport molaire entre a) et b) est compris entre 1:2 et 5:1.
